# EUROPÄISCHE PATENTSCHRIFT

(11) **EP 0 697 199 B1**
(45) Veröffentlichungstag und Bekanntmachung des Hinweises auf die Patenterteilung: **13.03.2002**
(21) Anmeldenummer: 95111621.9
(22) Anmeldetag: 24.07.1995
(51) Int. Cl.: A61B 18/14

(54) **Chirurgisches Bipolarinstrument**
Bipolar surgical instrument
Instrument chirurgical bipolaire

(30) Priorität: 18.08.1994 DE 4429260
(43) Veröffentlichungstag der Anmeldung: 21.02.1996
(73) Patentinhaber: Aesculap AG & Co. KG, 78532 Tuttlingen (DE)
(72) Erfinder: Keller, Anton, D 78589 Dürbheim (DE); Mayenberger, Rupert, D 78239 Rielasingen (DE); Rosenfelder, Georg, D 78054 Schwenningen-VS (DE)
(74) Vertreter: Hoeger, Stellrecht & Partner

(56) Entgegenhaltungen:
- EP-A- 0 188 701
- US-A- 3 970 088
- US-A- 4 014 343
- US-A- 4 074 718
- US-A- 4 593 691
- US-A- 5 196 007

## Beschreibung

Die Erfindung betrifft ein chirurgisches Bipolarinstrument mit einem rohrförmigen Gehäuse, in das ein mit zwei Elektroden versehener Elektrodenkopf unter Ausbildung einer elektrischen Verbindung zwischen den Elektroden und zwei im Gehäuse angeordneten Leitern lösbar einsteckbar ist.

Ein solches chirurgisches Bipolarinstrument ist beispielsweise bekannt aus der US-Patentschrift 4,043,342.

Dieses chirurgische Bipolarinstrument weist eine relativ große Halterung auf, an der ein Bipolarkopf lösbar befestigt werden kann. Durch diese relativ große Baugröße ist es auch möglich, eine lösbare Steckverbindung vorzusehen, bei der mehrere Stecker nebeneinander angeordnet werden.

Dies ist jedoch nicht möglich, wenn ein derartiges chirurgisches Instrument für die mikroinvasive Chirurgie verwendet werden soll, wenn also das Instrument durch Trokare hindurch in den Körper eingeführt werden soll und daher entsprechend raumsparend ausgestaltet werden muß.

Es ist Aufgabe der Erfindung, ein gattungsgemäßes chirurgisches Bipolarinstrument so auszubilden, daß es für den Einsatz in der mikroinvasiven Chirurgie besonders geeignet ist.

Diese Aufgabe wird bei einem chirurgischen Bipolarinstrument der eingangs beschriebenen Art erfindungsgemäß dadurch gelöst, daß ein Leiter durch einen leitenden, außenseitig elektrisch isolierten Rohrschaft und der andere durch einen im Rohrschaft elektrisch isoliert von diesem gelagerten Stab gebildet werden, daß eine Elektrode bei vollständig eingestecktem Elektrodenkopf an der Innenseite des Rohrschafts elektrisch leitend anliegende, federnd in radialer Richtung bewegbare, mit einem Vorsprung in einen Rücksprung der Innenwand des Rohrschafts oder des Stabs eingreifende und die Elektrode dadurch gegen eine axiale Verschiebung im rohrförmigen Gehäuse festlegende Einsteckelemente aufweist, daß Rohrschaft und Stab zwischen einer Verriegelungsposition und einer Freigabeposition in Längsrichtung relativ zueinander verschiebbar sind, wobei Rohrschaft und Stab in der Verriegelungsposition die Einsteckelemente mit dem Vorsprung in den Rücksprung eingreifend radial unverschiebbar zwischen sich einschließen, während sie in der Freigabeposition ein radiales Austreten des Vorsprungs aus dem Rücksprung zulassen, und daß der Stab in der Verriegelungsposition an der anderen Elektrode elektrisch leitend anliegt.

Durch die beschriebene Ausgestaltung wird es möglich, mit wenigen Teilen nicht nur einen raumsparenden Aufbau des Instruments zu erreichen, sondern auch eine lösbare und verriegelbare Verbindung zwischen dem Elektrodenkopf und dem Gehäuse vorzusehen.

Dazu werden einem Rohrschaft und einem darin angeordneten Stab zwei getrennte Aufgaben übertragen, nämlich einmal die Aufgabe einer lösbaren Halterung des Elektrodenkopfs nach Art einer Spannzange und zum anderen die elektrische Verbindung der beiden Elektroden des Elektrodenkopfs mit einer am anderen Ende des Instruments angeordneten elektrischen Versorgungseinheit. Ein wesentlicher Vorteil der Konstruktion liegt auch darin, daß der Elektrodenkopf nicht wie beim Stand der Technik nur lösbar und damit verlierbar in das Gehäuse eingesteckt wird, sondern daß eine Verriegelung in der eingesteckten Position erfolgt, so daß auch bei komplizierten Operationen nicht die Gefahr besteht, daß sich der Elektrodenkopf lockern oder gar von dem Gehäuse gelöst werden könnte.

Bei einer bevorzugten Ausführungsform ist vorgesehen, daß die Einsteckelemente als in Umfangsrichtung des Rohrschafts an diesem anliegende federnde Zungen oder Spannbacken ausgebildet sind.

Der Stab kann außenseitig einen elektrischen Isoliermantel tragen, vorzugsweise beispielsweise einen Schrumpfschlauch aus elektrisch isolierendem Kunststoffmaterial.

Besonders vorteilhaft ist es, wenn der Stab einen Abschnitt mit größerem Außendurchmesser aufweist, der in der Verriegelungsposition die Einsteckelemente radial unverschieblich gegenüber dem Rohrschaft festlegt. Dieser Abschnitt bildet einen Verriegelungskörper im Inneren des Rohrschafts, der die Einsteckelemente gegenüber der Innenwand des Rohrschafts unverschieblich festlegt und der bei Relativverschiebung des Stabs gegenüber dem Rohrschaft die freie radiale Bewegung der Einsteckelemente zuläßt, so daß die Vorsprünge der Einsteckelemente aus den entsprechenden Rücksprüngen austreten und in axialer Richtung verschoben werden können.

Besonders vorteilhaft ist es, wenn der Abschnitt mit größerem Außendurchmesser auf dem Stab durch ein Aufsteckteil aus elektrisch isolierendem Material gebildet wird. Dieses wird auf das Ende des Stabs aufgesteckt, umgibt diesen in diesem Bereich elektrisch isolierend und übt gleichzeitig die Funktion des Verriegelungskörpers aus.

Bei einer bevorzugten Ausführungsform ist vorgesehen , daß der Elektrodenkopf eine Hülse aus elektrisch isolierendem Material aufweist, in die einseitig ein Kontaktteil der anderen Elektrode und auf der anderen Seite der Stab eintauchen. Diese Hülse bildet somit eine Verbindungs- und Führungshülse, in der sich der Stab und die andere Elektrode treffen und dort eine elektrische Verbindung herstellen, wobei dieser Bereich nach außen hin elektrisch isoliert ist.

Insbesondere kann diese Hülse aus Keramik bestehen.

Es ist dabei weiterhin vorteilhaft, wenn die Hülse in einen aus Metall bestehenden Träger eingesetzt ist, der die eine Elektrode, das Einsteckelement sowie eine Verbindung zwischen Elektrode und Einsteckelementen umfaßt. Damit weist der Elektrodenkopf nur sehr wenige Teile auf, nämlich den Träger, die in diesen isolierend eingesetzte andere Elektrode und die Hülse zur Herstellung der Verbindung zwischen Stab und der anderen Elektrode.

Bei einer bevorzugten Ausführungsform ist vorgesehen, daß der Träger eine sich im wesentlichen über die Länge der einen Elektrode erstreckende, einseitig offene Ausnehmung zur Aufnahme eines Isolierkörpers umfaßt, in den die zweite Elektrode linienförmig überstehend eingebettet ist. Es ergibt sich dabei eine besonders günstige Konfiguration für die Elektroden, der Träger bildet nämlich eine großflächige, im Querschnitt U-förmige Elektrode aus, in die der Isolierkörper eingesetzt ist. Dieser nimmt seinerseits die zweite, schneidenförmig ausgebildete Elektrode auf, die linienförmig über den Isolierkörper vorsteht und somit eine linienförmige Elektrode bildet, die eine wesentlich kleinere Fläche aufweist als die durch den Träger gebildete flächige Elektrode.

Dabei kann vorgesehen sein, daß die zweite Elektrode längs einer Linie verläuft, die mindestens zwei unterschiedlich gerichtete Abschnitte aufweist, und daß der Isolierkörper parallel zu dieser Linie verlaufend ausgebildet ist. Dadurch ist es möglich, sehr unterschiedliche Konfigurationen der linienförmigen Elektrode zu verwirklichen, die den jeweiligen Einsatzzwecken angepaßt ist. Durch die leichte Auswechselbarkeit der Elektrodenköpfe ist es jederzeit möglich, Elektroden mit für den speziellen Einsatzzweck besonders geeigneter Linienführung zu verwenden.

Bei einer ersten Ausführungsform sind die beiden Abschnitte beispielsweise geradlinig ausgebildet und laufen von einem höchsten Punkt an ihren Enden zu einem gemeinsamen tiefsten Punkt. Man erhält damit eine abgeknickte Linie mit einer tiefsten Stelle etwa im Mittelbereich, so daß beispielsweise in diesem Bereich Gefäße lokalisiert werden können.

Bei einer anderen Ausführungsform kann vorgesehen sein, daß sich an einen höchsten Punkt der Linie ein zum freien Ende des Elektrodenkopfs abfallender Abschnitt anschließt, der in einen hakenförmig ansteigenden Abschnitt übergeht. Der Operateur hat damit die Möglichkeit, mit einer solchen Ausgestaltung ein gewünschtes Gewebeteil zu erfassen und gegebenenfalls aus seiner Körperposition zu verlagern, so daß dieses Gewebeteil isoliert geschnitten oder koaguliert werden kann.

Bei einer weiteren bevorzugten Ausführungsform kann vorgesehen sein, daß im Gehäuse ein dieses in Längsrichtung durchsetzender Strömungskanal angeordnet ist, der mit einem in die Umgebung austretenden Längskanal im Elektrodenkopf in Strömungsverbindung steht. Es wird dadurch möglich, durch diesen Strömungskanal eine Spül- oder Kühlflüssigkeit bis an den Elektrodenkopf heranzuführen und diese Flüssigkeit dann durch den Längskanal in die Umgebung des Elektrodenkopfs abzugeben, um dadurch eine Kühlung und Spülung zu erreichen.

Günstig ist es, wenn der Längskanal am vorderen Ende des Elektrodenkopfs austritt.

Bei einer speziellen Ausgestaltung ist dabei vorgesehen, daß der Längskanal durch aufeinanderliegende Rinnen in zwei Teilen des Elektrodenkopfs gebildet wird, insbesondere könne die zwei Teile die eine Elektrode und ein die andere Elektrode umgebender Isolierkörper sein.

Es ist vorteilhaft, wenn der Strömungskanal durch den Ringraum zwischen Rohrschaft und Stab gebildet wird, bei einer anderen Ausführungsform kann auch vorgesehen sein, daß der Strömungskanal im Inneren des Stabs verläuft.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht der wesentlichen Teile eines bipolaren Rohrschaftinstruments im Bereich des Elektrodenkopfs;
- Figur 2:: eine Schnittansicht längs Linie 2-2 bei verriegeltem Elektrodenkopf;
- Figur 3:: eine teilweise in Längsrichtung geschnittene Seitenansicht eines bevorzugten Ausführungsbeispiels eines Elektrodenkopfs;
- Figur 4:: eine Ansicht ähnlich Figur 3 bei einem anderen Ausführungsbeispiel eines Elektrodenkopfs;
- Figur 5:: eine Ansicht ähnlich Figur 3 bei einem weiteren bevorzugten Ausführungsbeispiel eines Elektrodenkopfs;
- Figur 6:: eine Draufsicht auf das vordere Ende des Elektrodenkopfs der Figur 2 in Richtung des Pfeils A in Figur 2 und
- Figur 7:: eine Schnittansicht längs Linie 7-7 in Figur 2.

Das in der Zeichnung dargestellte chirurgische Bipolarinstrument umfaßt einen länglichen, zylindrischen Rohrschaft 1 aus Metall, dessen Außenseite durch einen Isoliermantel 2 nach außen hin elektrisch isoliert ist. Der Isoliermantel 2 kann beispielsweise ein auf den Rohrschaft aufgeschrumpfter Kunststoffschlauch sein.

Im Inneren des Rohrschafts 1 ist in diesem zentral und im Abstand vom Rohrschaft 1 ein Stab 3 aus Metall angeordnet, der in dem Rohrschaft 1 durch nicht dargestellte Mittel zentral und längsverschieblich gelagert ist.

Wie dies bei chirurgischen Rohrschaftinstrumenten üblich und daher hier nicht eigens dargestellt ist, kann der Rohrschaft 1 starr mit einer Griffbranche verbunden sein, an der schwenkbar eine zweite Griffbranche angelenkt ist, die über eine Gelenkverbindung am Stab 3 angreift, so daß beim Verschwenken der Branchen gegeneinander der Stab 3 im Rohrschaft längsverschoben wird. Selbstverständlich sind auch andere Mechanismen der Längsverschiebung möglich.

Der Rohrschaft 1 und der Stab 3 bilden gemeinsam ein Gehäuse eines chirurgischen Bipolarinstruments, welches an seinem freien Ende einen Elektrodenkopf 4 trägt, der lösbar mit dem Rohrschaft 1 verbunden werden kann. Dieser Elektrodenkopf 4 umfaßt einen metallischen Träger 8, der seinerseits unterteilt ist in eine erste Elektrode 5, einen Einsteckabschnitt 6 und einen die Elektrode mit dem Einsteckabschnitt verbindenden Verbindungsteil 7.

Die erste Elektrode 5 ist im Querschnitt U-förmig ausgebildet und weist eine zwischen zwei parallelen Seitenwänden 9, 10 verlaufende, rinnenförmige Aufnahme 11 für einen Isolierkörper 12 auf, der in die Aufnahme 11 eingesetzt ist und diese vollständig ausfüllt.

Der Isolierkörper kann beispielsweise aus Keramik bestehen, in ihm ist eine parallel zu den Seitenwänden 9, 10 verlaufende, schneidenförmige zweite Elektrode 13 mittig zwischen den Seitenwänden 9 und 10 so angeordnet, daß der obere Rand 14 der Elektrode 13 linienförmig geringfügig über den Isolierkörper 12 hervorsteht.

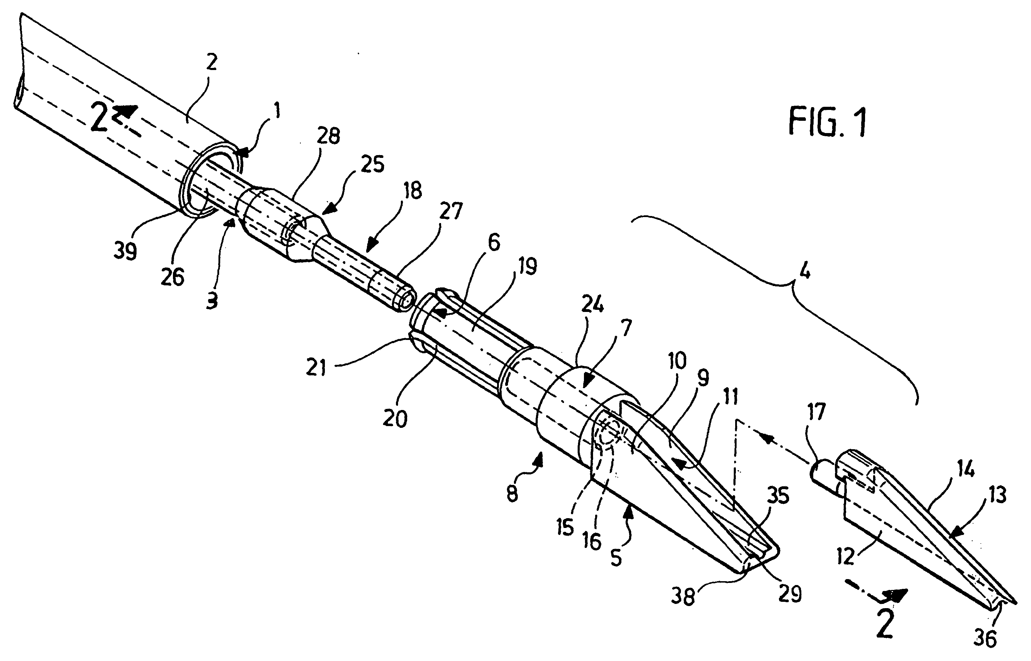

Bei dem in Figur 1 dargestellten Ausführungsbeispiel sind die Seitenwände 9, 10 vom hinteren Ende der ersten Elektrode 5 bis zu deren freiem Ende geradlinig abfallend ausgebildet, die gleiche Kontur nehmen der Isolierkörper 12 und die obere Kante 14 der zweiten Elektrode 13 an.

An das hintere Ende der Aufnahme 11 schließt sich der zylindrische Verbindungsteil 7 an, der eine durchgehende Bohrung 15 aufweist, in die eine die Bohrung 15 auskleidende Isolierhülse 16 aus Keramik eingesetzt ist. Diese durchsetzt den Verbindungsteil 7 über dessen gesamte Länge. In das der Aufnahme 11 benachbarte Ende der Isolierhülse 16 taucht ein mit der zweiten Elektrode 13 einstückig verbundener metallischer Kontaktstift 17 ein, in das gegenüberliegende Ende der Isolierhülse 16 wird das stiftförmig verjüngte Ende 18 des Stabs 3 eingeschoben.

An den Verlängerungsteil 7 schließen sich gemeinsam den Einsteckabschnitt ausbildende Einsteckelemente 19 an, die aus dem hülsenförmigen, sich an den Verbindungsteil 7 anschließenden Teil des Trägers durch Längsschlitze 20 entstehen. Diese Einsteckelemente 19 sind durch die Längsschlitze 20 radial federnd ausgebildet, wie dies bei Spannzangen oder Federzungen der Fall ist, an ihrem freien Ende tragen sie einen nach außen vorspringenden Ringwulst 21.

Der vorstehend beschriebene Elektrodenkopf 4 kann lösbar an dem aus Rohrschaft 1 und Stab 3 bestehenden Gehäuse festgelegt werden. Zu diesem Zweck befindet sich an der Innenseite 22 des Rohrschafts 1 eine umlaufende Umfangsnut 23, in die nach dem Einschieben der Einsteckelemente 19 der Ringwulst 21 an deren freiem Ende eintauchen kann. Die Einsteckelemente 19 liegen dann flächig an der Innenseite 22 an und stellen dort einen elektrischen Kontakt zwischen dem Träger 8 einerseits und dem Rohrschaft 1 andererseits her. Dieser Rohrschaft 1 legt sich dabei in eine Stufe 24 des Verlängerungsteils 7, so daß Verlängerungsteil 7 und Isoliermantel 2 bündig abschließen.

Um den Elektrodenkopf 4 in dieser Position zu sichern, trägt der Stab 3 in seinem Übergangsbereich zum stiftförmigen Ende 18 ein Aufsteckteil 25 aus elektrisch isolierendem Kunststoffmaterial, welches einen den Stab 3 umgebenden Isoliermantel 26 in Form eines aufgeschrumpften Kunststoffschlauchs umgibt und welches sich auf dem stiftförmigen Ende 18 bis an einen auf diesem festgelegten metallischen Ring 27 erstreckt, der bündig mit dem Aufsteckteil 25 abschließt.

Das Aufsteckteil 25 weist einen Bereich 28 mit größerem Außendurchmesser auf, dieser Bereich bildet einen Verriegelungskörper, der bei entsprechender Relativpositionierung des Stabs 3 und des Rohrschafts 1 die Einsteckelemente 19 radial nach außen drückt und dadurch den Ringwulst 21 in der Umfangsnut 23 festlegt. In dieser Position, die im folgenden als Verriegelungsposition bezeichnet wird, können sich also die Einsteckelemente 19 in radialer Richtung nicht mehr verschieben, so daß der Ringwulst 21 durch den Eingriff in die Umfangsnut 23 gleichzeitig auch den Elektrodenkopf 4 in axialer Richtung festlegt. Dabei taucht das stiftförmige Ende 18 des Stabs 3 in die Isolierhülse 16 ein und stellt dort durch Anlage am Kontaktstift 17 eine elektrische Verbindung zwischen dem Stab 3 einerseits und der zweiten Elektrode 13 andererseits her.

Um die Verriegelung des Elektrodenkopfs lösen zu können, muß entweder der Rohrschaft 1 oder der Stab 3 so weit nach hinten verschoben werden, daß der Bereich 28 eine radiale Bewegung der Einsteckelemente 19 nicht mehr behindert, so daß die Einsteckelemente 19 federnd nach außen bzw. nach innen gebogen werden können. Der Ringwulst 21 kann dann aus der Umfangsnut 23 austreten, und eine axiale Trennung des Elektrodenkopfs 4 von Rohrschaft und Stab ist möglich.

Bei dem dargestellten Ausführungsbeispiel greift der Ringwulst 21 in eine Umfangsnut 23 im Rohrschaft 1 ein, grundsätzlich wäre es auch möglich, an den Einsteckelementen einen in einen Rücksprung des Stabs eingreifenden Vorsprung vorzusehen, der durch den Rohrschaft am axialen Austreten aus dem Rücksprung gehindert wird. Die hier beschriebene Verbindung des Elektrodenkopfs mit dem Rohrschaft 1 und dem Stab 3 beruht auf dem Prinzip einer Spannzange, bei der die elastischen Einsteckelemente in der Freigabeposition radial verbiegbar sind, in der Verriegelungsposition jedoch nicht. Dieses Prinzip kann in unterschiedlichen Ausgestaltungen umgesetzt werden, die dem Fachmann an sich geläufig sind.

Es ist dabei wesentlich, daß im Rahmen der vorliegenden Konstruktion die Verbindungen zwischen dem Elektrodenkopf 4 einerseits und dem Rohrschaft 1 und dem Stab 3 andererseits nicht nur eine mechanische Verbindung des Elektrodenkopfs 4 hervorrufen, sondern gleichzeitig auch die elektrischen Verbindungen zwischen der ersten Elektrode 5 und der zweiten Elektrode 13 mit diesen beiden Teilen. Dadurch lassen sich mechanische und elektrische Verbindung durch ein Minimum an Teilen herstellen und trotzdem erhält man eine sichere und jederzeit wieder lösbare Verriegelung des Elektrodenkopfs.

Dies ist insbesondere unter dem Gesichtspunkt wichtig, daß Elektrodenköpfe 4 unterschiedlicher Konfigurationen eingesetzt werden sollen, so daß diese leicht auswechselbar sein müssen.

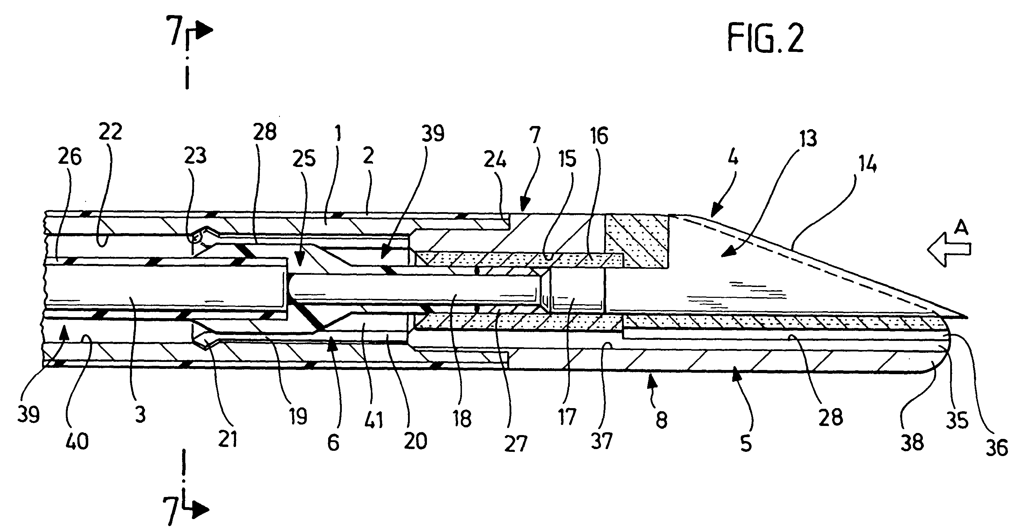

Bei dem Ausführungsbeispiel gemäß Figur 2 verläuft die obere Kante 14 der zweiten Elektrode 13 vom gehäuseseitigen zum freien Ende hin geradlinig abfallend, wie dies aus der Darstellung der Figuren 1 und 2 ersichtlich wird.

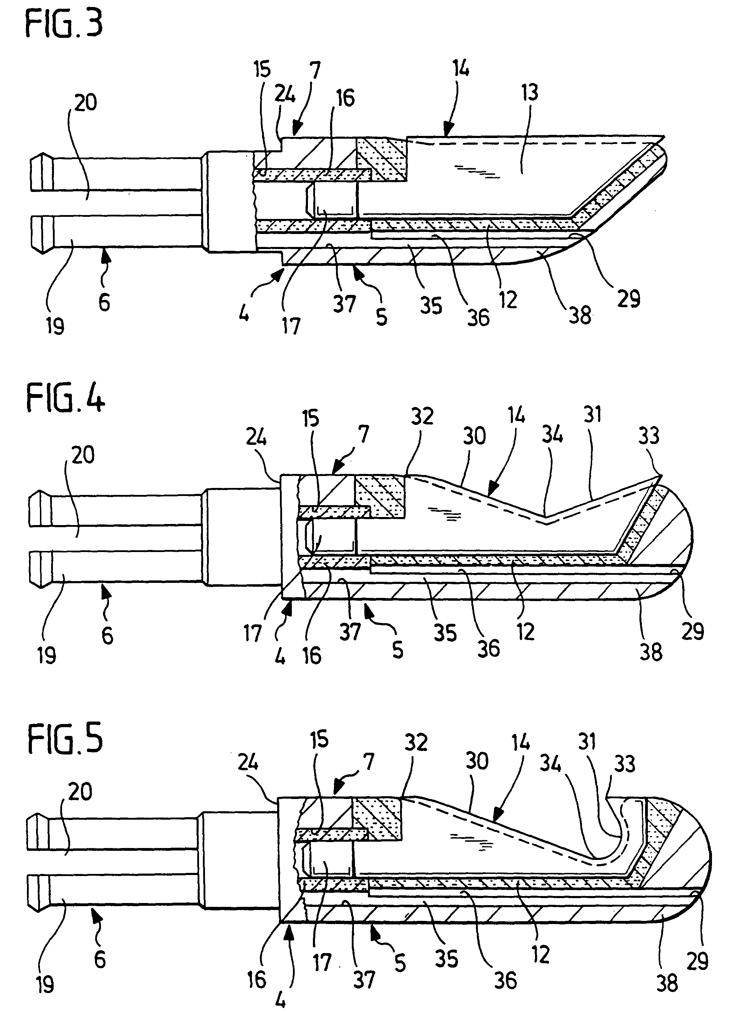

Bei dem ansonsten gleich aufgebauten Ausführungsbeispiel der Figur 3, bei dem gleiche Teile daher dieselben Bezugszeichen tragen, verläuft die obere Kante 14 dagegen parallel zum Boden 29 der Aufnahme 11, das heißt parallel zur Längsachse des Instruments.

Bei dem Ausführungsbeispiel der Figur 4, das sich nur durch den Verlauf der oberen Kante 14 von den bisher erörterten Ausführungsbeispielen unterscheidet, ist die obere Kante 14 in zwei geradlinige Abschnitte 30, 31 unterteilt, die von einem höheren Punkt 32 beziehungsweise 33 an den Enden der zweiten Elektrode zu einem zwischen den Enden angeordneten, tiefsten Punkt 34 hin abfallend.

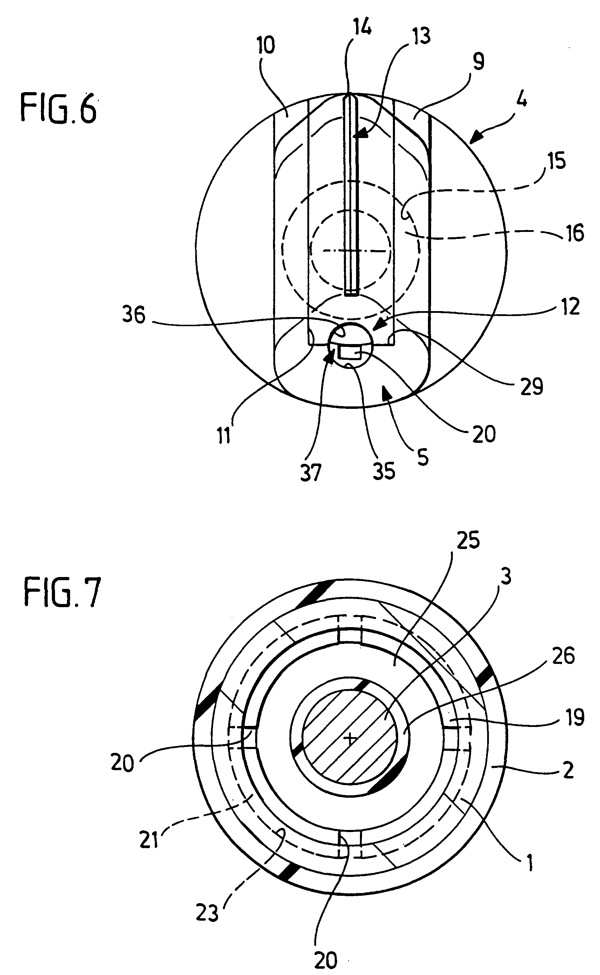

Bei dem Ausführungsbeispiel der Figur 5 schließlich, das bis auf den Verlauf der oberen Kante 14 wieder gleich ausgebildet ist, weist diese obere Kante einen vom hinteren höchsten Punkt 32 zu einem tiefsten Punkt 34 abfallenden geradlinigen Abschnitt 30 und einen daran anschließenden, bogenförmigen Abschnitt 31 auf, so daß die zweite Elektrode insgesamt die Form eines Hakens erhält.

Selbstverständlich sind auch andere Verläufe der oberen Kante 14 möglich, und es ist für den Operateur daher von großem Vorteil, daß er die verschieden geformten Elektrodenköpfe schnell auswechseln und am selben Instrument einsetzen kann, es genügt nämlich dazu, den Stab 3 gegenüber dem Rohrschaft 1 in Längsrichtung in die Freigabestellung zu verschieben und den Elektrodenkopf 4 in Längsrichtung von dem Instrument abzuziehen. In umgekehrter Richtung kann ein neu aufgestecker Elektrodenkopf wieder verriegelt werden, wobei dabei gleichzeitig auch die elektrischen Verbindungen hergestellt werden.

Im Boden 29 der Aufnahme 11 befindet sich eine in Längsrichtung durchlaufende Rinne 35, die zusammen mit einer sie abdeckenden Rinne 36 in der Unterseite des Isolierkörpers 12 einen geschlossenen Längskanal 37 ausbildet, der den Elektrodenkopf 4 in Längsrichtung durchsetzt und der am vorderen Ende 38 des Elektrodenkopfs 4 austritt.

Durch diesen Längskanal 37 wird eine Verbindung zwischen dem Ringkanal 39 im Gehäuse und der Umgebung hergestellt, der begrenzt wird einerseits durch den Stab 3 mit dem Aufsteckteil 25 und andererseits durch den Rohrschaft 1. Dieser Ringkanal 39 wird durch den Bereich 28 mit vergrößertem Außendurchmesser des Aufsteckteils 25 und durch die von diesem gegen die Innenseite 22 des Rohrschafts 1 gedrückten Einsteckelemente 19 in einen stromabwärts gelegenen Teil 40, der an den Elektrodenkopf 4 angrenzt, und einen stromaufwärts gelegenen Teil 41 getrennt. Beide Teile stehen durch die Längsschlitze 20 zwischen den Einsteckelementen 19 miteinander in Verbindung, so daß durch den Ringkanal 39 eine Spül- und Kühlflüssigkeit vom entfernten Ende des Rohrschafts 1 zum Elektrodenkopf 4 herangeführt werden kann; diese Flüssigkeit tritt dann durch den Längskanal 37 am vorderen Ende 38 des Elektrodenkopfs 4 aus und spült und kühlt den Eingriffsbereich der Elektroden.

Der Längskanal 37 kann auch durch seitliche, in der Zeichnung nicht dargestellte zusätzliche Öffnungen Flüssigkeit seitlich aus dem Elektrodenkopf abgeben, hier sind verschiedene Muster des Austritts möglich.

Bei einem in der Zeichnung nicht dargestellten, abgewandelten Beispiel kann auch vorgesehen sein, daß der Strömungskanal im Inneren des Rohrschafts nicht durch den Ringkanal 39 gebildet wird, sondern daß im Inneren des Stabs 3 ein entsprechender Strömungskanal angeordnet ist, der in Strömungsverbindung steht mit dem Längskanal 37 oder mit einem Längskanal im Elektrodenkopf, der beispielsweise durch den Kontaktstift 17 geführt ist.

## Patentansprüche

1. Chirurgisches Bipolarinstrument mit einem rohrförmigen Gehäuse, in das ein mit zwei Elektroden versehener Elektrodenkopf unter Ausbildung einer elektrischen Verbindung zwischen den Elektroden und zwei im Gehäuse angeordneten Leitern lösbar einsteckbar ist, **dadurch gekennzeichnet, daß** ein Leiter durch einen leitenden, außenseitig elektrisch isolierten Rohrschaft (1) und der andere durch einen im Rohrschaft (1) elektrisch isoliert von diesem gelagerten Stab (3) gebildet werden, daß eine Elektrode (5) bei vollständig eingestecktem Elektrodenkopf (4) an der Innenseite des Rohrschafts (1) elektrisch leitend anliegende, federnd in radialer Richtung bewegbare, mit einem Vorsprung (21) in einen Rücksprung (23) der Innenwand des Rohrschafts (1) oder des Stabs eingreifende und die eine Elektrode (5) dadurch gegen eine axiale Verschiebung im rohrförmigen Gehäuse festlegende Einsteckelemente (19) aufweist, daß Rohrschaft (1) und Stab (3) zwischen einer Verriegelungsposition und einer Freigabeposition in Längsrichtung relativ zueinander verschiebbar sind, wobei Rohrschaft (1) und Stab (3) in der Verriegelungsposition die Einsteckelemente (19) mit dem Vorsprung (21) in den Rücksprung (23) eingreifend radial unverschiebbar zwischen sich einschließen, während sie in der Freigabeposition ein radiales Austreten des Vorsprungs (21) aus dem Rücksprung (23) zulassen, und daß der Stab (3) in der Verriegelungsposition an der anderen Elektrode (13) elektrisch leitend anliegt.

2. Chirurgisches Bipolarinstrument nach Anspruch 1, **dadurch gekennzeichnet, daß** die Einsteckelemente (19) als in Umfangsrichtung des Rohrschafts (1) an diesem anliegende federnde Zungen oder Spannbacken ausgebildet sind.

3. Chirurgisches Bipolarinstrument nach Anspruch 1 oder 2, **dadurch gekennzeichnet, daß** der Stab (3) außenseitig einen elektrischen Isoliermantel (26, 25) trägt.

4. Chirurgisches Bipolarinstrument nach einem der voranstehenden Ansprüche, **dadurch gekennzeichnet, daß** der Stab (3) einen Abschnitt (28) mit größerem Außendurchmesser aufweist, der in der Verriegelungsposition die Einsteckelemente (19) radial unverschieblich gegenüber dem Rohrschaft (1) festlegt.

5. Chirurgisches Bipolarinstrument nach Anspruch 4, **dadurch gekennzeichnet, daß** der Abschnitt (28) mit größerem Außendurchmesser durch ein Aufsteckteil (25) aus elektrisch isolierendem Material gebildet wird.

6. Chirurgisches Bipolarinstrument nach einem der voranstehenden Ansprüche, **dadurch gekennzeichnet, daß** der Elektrodenkopf (4) eine Hülse (16) aus elektrisch isolierendem Material aufweist, in die einseitig ein Kontaktteil (17) der anderen Elektrode (13) und auf der anderen Seite der Stab (3) eintauchen.

7. Chirurgisches Bipolarinstrument nach Anspruch 6, **dadurch gekennzeichnet, daß** die Hülse (16) aus Keramik besteht.

8. Chirurgisches Bipolarinstrument nach einem der Ansprüche 6 oder 7, **dadurch gekennzeichnet, daß** die Hülse (16) in einen aus Metall bestehenden Träger (8) eingesetzt ist, der die eine Elektrode (5), die Einsteckelemente (19) sowie eine Verbindung (7) zwischen Elektrode (5) und Einsteckelementen (19) umfaßt.

9. Chirurgisches Bipolarinstrument nach Anspruch 8, **dadurch gekennzeichnet, daß** der Träger (8) eine sich im wesentlichen über die Länge der einen Elektrode (5) erstreckende, einseitig offene Ausnehmung (11) zur Aufnahme eines Isolierkörpers (12) umfaßt, in den die andere Elektrode (13) linienförmig überstehend eingebettet ist.

10. Chirurgisches Bipolarinstrument nach Anspruch 9, **dadurch gekennzeichnet, daß** die andere Elektrode (13) längs einer Linie (14) verläuft, die mindestens zwei unterschiedlich gerichtete Abschnitte (30, 31) aufweist, und daß der Isolierkörper (12) parallel zu dieser Linie (14) verlaufend ausgebildet ist.

11. Chirurgisches Bipolarinstrument nach Anspruch 10, **dadurch gekennzeichnet, daß** die beiden Abschnitte (30, 31) geradlinig ausgebildet sind und von einem höchsten Punkt (32, 33) an ihren Enden zu einem gemeinsamen tiefsten Punkt (34) laufen.

12. Chirurgisches Bipolarinstrument nach Anspruch 10, **dadurch gekennzeichnet, daß** sich an einen höchsten Punkt (32) der Linie (14) ein zum freien Ende des Elektrodenkopfs (4) abfallender Abschnitt (30) anschließt, der in eine hakenförmig ansteigenden Abschnitt (31) übergeht.

13. Chirurgisches Bipolarinstrument nach einem der voranstehenden Ansprüche, **dadurch gekennzeichnet, daß** im Gehäuse ein dieses in Längsrichtung durchsetzender Strömungskanal vorgesehen ist, der mit einem in die Umgebung austretenden Längskanal (37) im Elektrodenkopf (4) in Strömungsverbindung steht.

14. Chirurgisches Bipolarinstrument nach Anspruch 13, **dadurch gekennzeichnet, daß** der Längskanal (37) am vorderen Ende (38) des Elektrodenkopfs (4) austritt.

15. Chirurgisches Bipolarinstrument nach einem der Ansprüche 13 oder 14, **dadurch gekennzeichnet, daß** der Längskanal (37) durch aufeinanderliegende Rinnen (35, 36) in zwei Teilen des Elektrodenkopfs (4) gebildet wird.

16. Chirurgisches Bipolarinstrument nach Anspruch 15, **dadurch gekennzeichnet, daß** die zwei Teile die eine Elektrode (5) und ein die andere Elektrode (13) umgebender Isolierkörper (12) sind.

17. Chirurgisches Bipolarinstrument nach einem der Ansprüche 13 bis 16, **dadurch gekennzeichnet, daß** der Strömungskanal durch den Ringraum (39) zwischen Rohrschaft (1) und Stab (3) gebildet wird.

18. Chirurgisches Bipolarinstrument nach einem der Ansprüche 13 bis 16, **dadurch gekennzeichnet, daß** der Strömungskanal im Inneren des Stabs (3) verläuft.

## Claims

1. A bipolar surgical instrument with a tubular housing, into which an electrode head provided with two electrodes is releasably insertable, forming an electrical connection between the electrodes and two conductors arranged within the housing, **characterised in that** one conductor is in the form of a conducting tubular shaft (1) electrically insulated on the outside, and the other is in the form of a rod (3) positioned in the tubular shaft (1) so as to be electrically insulated therefrom, **in that**, when the electrode head (4) is fully inserted, one electrode (5) exhibits insertion members (19) which lie adjacent to the inside of the tubular shaft (1) so as to be electrically conductive and are elastically movable in the radial direction, and which with a projection (21) engage into a recess (23) in the internal wall of the tubular shaft (1) or in the rod and by this means secure the one electrode (5) against axial displacement within the tubular housing, **in that** tubular shaft (1) and rod (3) are displaceable in a longitudinal direction relative to one another between a locking position and a release position, in the locking position tubular shaft (1) and rod (3) enclosing the insertion members (19) engaging with the projection (21) in the recess (23) so as to be radially non-displaceable therebetween, whereas in the release position they permit radial disengagement of the projection (21) from the recess (23), and **in that**, in the locking position, the rod (3) is adjacent to the other electrode (13) so as to be electrically conductive.

2. A bipolar surgical instrument according to Claim 1, **characterised in that** the insertion members (19) are in the form of resilient tongues or fastening lugs adjacent to the tubular shaft (1) in the circumferential direction thereof.

3. A bipolar surgical instrument according to Claim 1 or 2, **characterised in that** the rod (3) has an electrically insulating covering (26, 25) on the outside.

4. A bipolar surgical instrument according to one of the preceding claims, **characterised in that** the rod (3) exhibits a section (28) of larger outer diameter which, in the locking position, secures the insertion members (19) radially non-displaceably relative to the tubular shaft (1).

5. A bipolar surgical instrument according to Claim 4, **characterised in that** the section (28) of larger outer diameter is in the form of a slip-on part (25) made of an electrically insulating material.

6. A bipolar surgical instrument according to one of the preceding claims, **characterised in that** the electrode head (4) has a sleeve (16) made of an electrically insulating material, into which on one side a contact part (17) of the other electrode (13) and on the other side a rod (3) are inserted.

7. A bipolar surgical instrument according to Claim 6, **characterised in that** the sleeve (16) consists of ceramic.

8. A bipolar surgical instrument according to one of Claims 6 or 7, **characterised in that** the sleeve (16) is inserted into a metal carrier (8) which comprises the one electrode (5), the insertion members (19), and a connector (7) between electrode (5) and insertion members (19).

9. A bipolar surgical instrument according to Claim 8, **characterised in that** the carrier (8) exhibits a recess (11) open on one side and extending substantially over the length of the one electrode (5), for accommodating an insulator (12) into which the other electrode (13) is embedded so as to project linearly.

10. A bipolar surgical instrument according to Claim 9, **characterised in that** the other electrode (13) follows a course along a line (14) which has at least two differently orientated sections (30, 31) and **in that** the insulator (12) is constructed so as to follow a course parallel to this line (14).

11. A bipolar surgical instrument according to Claim 10, **characterised in that** the two sections (30, 31) are straight and follow a course from a highest point (32, 33) to a common lowest point (34).

12. A bipolar surgical instrument according to Claim 10, **characterised in that** a section (30) which slopes downwards towards the free end of the electrode head (4) adjoins a highest point (32) of the line (14), the said section (30) changing into a section (31) which slopes upwards in the shape of a hook.

13. A bipolar surgical instrument according to one of the preceding claims, **characterised in that** a flow channel is provided in the housing, through which it passes in the longitudinal direction, the said flow channel being in flow connection with a channel (37) in the electrode head (4) which opens into the environment.

14. A bipolar surgical instrument according to Claim 13, **characterised in that** the longitudinal channel (37) opens on the anterior end of the electrode head (4).

15. A bipolar surgical instrument according to one of Claims 13 or 14, **characterised in that** the longitudinal channel (37) is formed by grooves (35, 36) superimposed on one another in two parts of the electrode head (4).

16. A bipolar surgical instrument according to Claim 15, **characterised in that** the two parts are insulating bodies (12), one surrounding the one electrode (5) and one surrounding the other electrode (13).

17. A bipolar surgical instrument according to one of the Claims 13 to 16, **characterised in that** the flow channel is formed by the annular space (39) between tubular shaft (1) and rod (3).

18. A bipolar surgical instrument according to one of the Claims 13 to 16, **characterised in that** the flow channel runs through the inside of the rod (3).

## Revendications

1. Instrument chirurgical bipolaire, comportant un boîtier tubulaire dans lequel peut venir s'enficher de façon détachable une tête pourvue de deux électrodes en établissant une connexion électrique entre les électrodes et deux conducteurs agencés dans le boîtier, **caractérisé en ce qu'**un conducteur est formé par une tige tubulaire (1) conductrice électriquement isolée du côté extérieur et l'autre conducteur est formé par une barre (3) montée dans la tige tubulaire (1) de façon électriquement isolée de celle-ci, **en ce qu'**une électrode (5) comprend des éléments enfichables (19) qui s'appliquent, dans l'état complètement enfiché de la tête à électrodes (4), contre le côté intérieur de la tige tubulaire (1) en établissant une conduction électrique, qui sont élastiquement mobiles en direction radiale, qui s'engagent par une saillie (21) dans un retrait (23) de la paroi intérieure de la tige tubulaire (1) ou de la barre et qui fixent ainsi l'une des électrodes (5) à l'encontre d'une translation axiale dans le boîtier tubulaire, **en ce que** la tige tubulaire (1) et la barre (3) sont mobiles en translation l'une par rapport à l'autre en direction longitudinale entre une position de verrouillage et une position de libération, la tige tubulaire (1) et la barre (3) enfermant entre elles de façon radialement immobile, dans la position de verrouillage, les éléments enfichables (19) par engagement de la saillie (21) dans le retrait (23), tandis que dans la position de libération elles permettent une sortie radiale de la saillie (21) hors du retrait (23), et **en ce que** dans la position de verrouillage la barre (3) s'applique contre l'autre électrode (13) en établissant une conduction électrique.

2. Instrument bipolaire chirurgical selon la revendication 1, **caractérisé en ce que** les éléments enfichables (19) sont réalisés sous la forme de languettes ou de mâchoires de serrage élastiques qui s'appliquent contre la tige tubulaire (1) en direction périphérique de celle-ci.

3. Instrument bipolaire chirurgical selon l'une ou l'autre des revendications 1 et 2, **caractérisé en ce que** la barre (3) porte du côté extérieur une enveloppe d'isolation électrique (26, 25).

4. Instrument bipolaire chirurgical selon l'une quelconque des revendications précédentes, **caractérisé en ce que** la barre (3) comprend un tronçon (28) de diamètre extérieur plus grand qui fixe dans la position de verrouillage les éléments enfichables (19) de façon radialement immobile par rapport à la tige (1).

5. Instrument bipolaire chirurgical selon la revendication 4, **caractérisé en ce que** le tronçon (28) de plus grand diamètre extérieur est formé par une partie de coiffe (25) en matériau électriquement isolant.

6. Instrument bipolaire chirurgical selon l'une quelconque des revendications précédentes, **caractérisé en ce que** la tête à électrodes (4) comprend une douille (16) en matériau électriquement isolant dans laquelle viennent plonger sur un côté la partie de contact (17) de l'autre électrode (13) et sur l'autre côté la barre (3).

7. Instrument bipolaire chirurgical selon la revendication 6, **caractérisé en ce que** la douille (16) est constituée en céramique.

8. Instrument bipolaire chirurgical selon l'une quelconque des revendications 6 et 7, **caractérisé en ce que** la douille (16) est mise en place dans un support (8) constitué en métal qui comprend l'une des électrodes (5), les éléments enfichables (19), ainsi qu'une connexion (7) entre l'électrode et les éléments enfichables (19).

9. Instrument bipolaire chirurgical selon la revendication 8, **caractérisé en ce que** le support (8) comprend un évidement (11) s'étendant sensiblement sur la longueur de l'une des électrodes (5), ouvert sur un côté et destiné à recevoir un corps d'isolation (12) dans lequel l'autre électrode (13) est noyée de manière à former une saillie linéaire.

10. Instrument bipolaire chirurgical selon la revendication 9, **caractérisé en ce que** l'autre électrode (13) s'étend le long d'une ligne (14) qui comprend au moins deux tronçons (30, 31) à orientations différentes, et **en ce que** le corps d'isolation (12) est réalisé de manière à s'étendre parallèlement à cette ligne (14).

11. Instrument bipolaire chirurgical selon la revendication 10, **caractérisé en ce que** les deux tronçons (30, 31) sont réalisés rectilignes et s'étendent à partir d'un point (32, 33) de hauteur maximum au niveau de leurs extrémités jusqu'à un point commun (34) de hauteur minimum.

12. Instrument bipolaire chirurgical selon la revendication 10, **caractérisé en ce qu'**un tronçon (30) descendant vers l'extrémité libre de la tête à électrodes (4) se raccorde à un point (32) de hauteur maximum de la ligne (14) et qui se transforme en un tronçon (31) montant en forme de crochet.

13. Instrument bipolaire chirurgical selon l'une quelconque des revendications précédentes, **caractérisé en ce qu'**il est prévu dans le boîtier un canal d'écoulement qui le traverse en direction longitudinale et qui est en communication d'écoulement avec un canal longitudinal (37) dans la tête à électrodes (4), qui sort vers l'environnement.

14. Instrument bipolaire chirurgical selon la revendication 13, **caractérisé en ce que** le canal longitudinal (37) sort à l'extrémité antérieure (38) de la tête à électrodes (4).

15. Instrument bipolaire chirurgical selon l'une ou l'autre des revendications 13 et 14, **caractérisé en ce que** le canal longitudinal (37) est formé par des rainures superposées (35, 36) dans deux parties de la tête à électrodes (4).

16. Instrument bipolaire chirurgical selon la revendication 15, **caractérisé en ce que** les deux parties sont formées par une électrode (5) et par un corps d'isolation (12) entourant l'autre électrode (13).

17. Instrument bipolaire chirurgical selon l'une quelconque des revendications 13 à 16, **caractérisé en ce que** le canal d'écoulement est formé par la chambre annulaire (39) entre la tige tubulaire (1) et la barre (3).

18. Instrument bipolaire chirurgical selon l'une quelconque des revendications 13 à 16, **caractérisé en ce que** le canal d'écoulement s'étend à l'intérieur de la barre (3).
